Europäisches Patentamt

European Patent Office

Office européen des brevets

(19)

(11) **EP 1 405 157 B1**

(12) **EUROPÄISCHE PATENTSCHRIFT**

(45) Veröffentlichungstag und Bekanntmachung des Hinweises auf die Patenterteilung:
**12.04.2006 Patentblatt 2006/15**

(21) Anmeldenummer: **01913876.7**

(22) Anmeldetag: **13.03.2001**

(51) Int Cl.:
***G06F 3/00*** (2006.01)

(86) Internationale Anmeldenummer:
**PCT/EP2001/002831**

(87) Internationale Veröffentlichungsnummer:
**WO 2002/065898 (29.08.2002 Gazette 2002/35)**

(54) **VORRICHTUNG UND VERFAHREN ZUR BLICKRICHTUNGSBESTIMMUNG BEZÜGLICH EINES FESTEN REFERENZKOORDINATENSYSTEMS**

DEVICE AND METHOD FOR DETERMINING THE VIEWING DIRECTION IN TERMS OF A FIXED REFERENCE CO-ORDINATES SYSTEM

DISPOSITIF ET PROCEDE POUR DETERMINER LA DIRECTION DU REGARD RELATIVEMENT A UN SYSTEME FIXE DE COORDONNEES DE REFERENCE

(84) Benannte Vertragsstaaten:
**DE FR GB**

(30) Priorität: **19.02.2001 DE 10107685**

(43) Veröffentlichungstag der Anmeldung:
**07.04.2004 Patentblatt 2004/15**

(73) Patentinhaber: **Fraunhofer-Gesellschaft zur Förderung der angewandten Forschung e.V.
80686 München (DE)**

(72) Erfinder:
- **WIEBE, Peter
  58285 Gevelsberg (DE)**
- **FAKESCH, Uwe
  40477 Düsseldorf (DE)**
- **NEHRIG, Oliver
  47441 Moers (DE)**

(74) Vertreter: **Schoppe, Fritz
Patentanwälte
Schoppe, Zimmermann, Stöckeler & Zinkler
Postfach 246
82043 Pullach bei München (DE)**

(56) Entgegenhaltungen:
EP-A- 0 468 340          EP-A- 0 578 908
WO-A-98/11528          US-A- 4 582 403
US-A- 5 517 021

- TAKAMI O ET AL: "Computer interface to use head and eyeball movement for handicapped people" SYSTEMS, MAN AND CYBERNETICS, 1995. INTELLIGENT SYSTEMS FOR THE 21ST CENTURY., IEEE INTERNATIONAL CONFERENCE ON VANCOUVER, BC, CANADA 22-25 OCT. 1995, NEW YORK, NY, USA,IEEE, US, 22. Oktober 1995 (1995-10-22), Seiten 1119-1123, XP010194425 ISBN: 0-7803-2559-1

EP 1 405 157 B1

**Beschreibung**

Die vorliegende Erfindung bezieht sich auf Vorrichtungen und Verfahren zur Blickrichtungsbestimmung und insbesondere solche Vorrichtungen und Verfahren, die eine absolute Blickrichtungsbestimmung, d.h. eine Blickrichtungsbestimmung bezüglich eines festen Referenzkoordinatensystems ermöglichen.

In der heutigen Zeit, die mehr und mehr durch Technologie und Automatisierung dominiert wird, existieren zahlreiche Systeme, die auf eine Interaktion zwischen Menschen und Maschinen zurückgreifen. Alle derartigen Systeme benötigen eine Schnittstelle zwischen dem Mensch als Benutzer und der Maschine, um Informationen zwischen dem Benutzer und dem technischen System transportieren zu können. Typische solche Schnittstellen in Verbindung mit einem Computer sind Tastatur, Maus, Trackball und dergleichen.

Die oben genannten Schnittstellen dienen zur Eingabe von Informationen per Hand. Derartige Schnittstellen können jedoch unter verschiedenen speziellen Bedingungen und physikalischen Beschränkungen problematisch sein. Für einen Benutzer, der eine Textverarbeitung über Tastatur durchführt, kann es beispielsweise hinderlich sein, einen Cursor auf dem Bildschirm per herkömmlicher Maus zu bewegen, da er dann eine Hand von der Tastatur nehmen muß. Ferner stehen eine oder beide Hände bei computergesteuerten Maschinen und Vorrichtungen häufig nicht zur Verfügung, um Eingaben durchzuführen. Darüber hinaus sind die oben genannten Schnittstellen bei behinderten Personen problematisch.

Wie bei P. Wiebe: "Grundlage der Informationsübertragung mit Biosignalen für den Aufbau von technischen Kommunikationshilfen bei behinderten Menschen", Biomedical Engineering, Band 45, Heft 1-2, 2000, Seiten 14-19, beschrieben ist, basieren Mensch-Maschine-Schnittstellen stets auf willentlich beeinflußbaren Handlungen bzw. Biosignalen. Solche Handlungen können z.B. in der Erzeugung akustischer Signale, elektrophysiologischer Signale bzw. einer Bewegung von Körperteilen bestehen.

Es existieren bereits Vorschläge für Mensch-Maschine-Schnittstellen, die Informationseingaben in ein System ermöglichen, ohne zu diesem Zweck Hände oder Sprache zu benötigen. Beispielsweise existieren Systeme, bei denen eine Kopfbewegung erfaßt wird, um basierend auf der Bewegung des Kopfes eine Bewegung eines Cursors auf einem Bildschirm zu bewirken. Andere Systeme verwenden die menschliche Blickrichtung, um die jeweilige Mensch-Maschine-Schnittstelle zu steuern. Hinweise auf derartige Systeme bzw. technische Grundlagen dafür sind den folgenden Fundstellen zu entnehmen:

LaCourse J.R., u.a., "An Eye Movement Communication-Control System for the Disabled", IEEE Transaction on Biomedical Engineering, 1990, 37(12), Seiten 1215-1220;

Hutten H. u.a., "Hochauflösendes Verfahren zur Kontrolle der Augenstellung", Biomedizinische Technik, Band 43, Ergänzungsband 1, 1998, Seiten 108 und 109;

G. Wießpeiner u.a., "Eye-Writer", Biomedizinische Technik, Band 43, Ergänzungsband 2, 1998, Seiten 158 bis 161;

D.W. Pathmore u.a., "Towards an EOG-Based Eye Tracker for Computer Control", Third Annual ACM Conference on Assistive Technologies, 1998, Seiten 197-203;

P. Wiebe u.a.: Biosignalverarbeitung des menschlichen Elektrookulogramms (EOG) zur Steuerung von Computer-Eingabemedien für gelähmte Menschen", Biomedizinische Technik/Biomedical Engineering, Band 45, Ergänzungsband 1, 2000, S. 184-185; und

D.R. Asche u.a., "A Three-Electrode EOG for Use as a Communication Interface for the Non-Vocal, Physically Handicapped", 29th ACEMB, Sheraton-Boston, Massachusetts, 6. bis 10. Nov. 1976, Seite 2.

Alle diese bekannten Systeme verwenden die Augenblickrichtung eines Benutzers relativ zum Kopf des Benutzers, also eine relative Augenbewegung. Diese Augenbewegung kann erfaßt werden, da das Auge ein elektrisches Dipolfeld erzeugt, das starr mit dem Auge bzw. der Augenbewegung gekoppelt ist, so daß die Augenbewegungen gleichzeitig die Lage des Dipolfelds ändern. Eine Augenbewegung stellt ein zum großen Teil willentlich reproduzierbares und steuerbares Biosignal dar, das meßtechnisch erfaßt werden kann, um basierend darauf beispielsweise einen Cursor auf einem Bildschirm zu bewegen. Das über das Dipolfeld des Auges erfaßte Biosignal wird als Elektrookulogramm (EOG) bezeichnet.

Eine vorteilhafte Ausgestaltung einer Vorrichtung zur Aufnahme von Elektrookulogrammen ist in der oben genannten Schrift "Biosignalverarbeitung des menschlichen Elektrookulogramms (EOG) zur Steuerung von Computer-Eingabemedien für gelähmte Menschen" von P. Wiebe beschrieben. Dabei wird eine Vorrichtung verwendet, die durch einen Benutzer wie eine Brille getragen werden kann, wobei drei Elektroden vorgesehen sind, um Augenvertikalbewegungen und Augenhorizontalbewegungen, d.h. die Relativblickrichtung des Benutzers, sowie Lidschläge erfassen zu können. Basierend auf den so erfaßten Elektrookulogrammen werden dann Mensch-Maschine-Interaktionen durchgeführt, beispielsweise eine Bewegung eines Cursors auf einem Bildschirm oder eine Eingabe, die bei herkömmlichen Schnittstellen durch einen "Mausklick" beispielsweise mit der linken Computermaustaste durchgeführt wird.

Ferner sind aus dem Stand der Technik Sensoren für Trägheitsnavigationssysteme bekannt, die die Ermittlung der Position eines Objekts im Raum ermöglichen. Diesbezüglich sei beispielsweise auf C.Lemair u.a., "Surface Micromachined Sensors for Vehicle Navigation Systems", Advanced Microsystems for Automotive Application 98 (Berlin), Springer Verlag, 1998, Seiten 112 bis 133, und J. Söderkvist, "Micromachined Gyroscopes", Sensors and Actuators A, 43, 1994, Seiten 65 bis 71, verwiesen.

Aus der US-A-5517021 ist ein System zur Erfassung von Elektrookulogrammen zur relativen Blickrichtungsbestimmung bekannt.

Die Aufgabe der vorliegenden Erfindung besteht darin, Vorrichtungen und Verfahren zu schaffen, die eine flexible und für einen Benutzer bequeme Schnittstelle zwischen Mensch und Maschine ermöglichen.

Diese Aufgabe wird durch eine Vorrichtung nach Anspruch 1 und ein Verfahren nach Anspruch 6 gelöst.

Die vorliegende Erfindung schafft somit Verfahren und Vorrichtungen, die eine Bestimmung der menschlichen Blickrichtung bezüglich eines festen Referenzkoordinatensystems ermöglichen, wobei diese Blickrichtung im folgenden als absolute Blickrichtung bezeichnet wird, im Gegensatz zur relativen Blickrichtung der Augen bezüglich des Kopfs des Benutzers. Der vorliegenden Erfindung liegt die Erkenntnis zugrunde, daß diese absolute Blickrichtung sowohl von der relativen Augenstellung bezüglich des Kopfs als auch der absoluten Kopfposition, d.h. der Kopfposition relativ zu dem festen Referenzkoordinatensystem, abhängt. Um somit die absolute Blickrichtung bestimmen zu können, muß zum einen die relative Blickrichtung erfaßt werden, was erfindungsgemäß vorzugsweise durch Erfassung eines Elektrookulogramms erfolgt. Zum anderen muß die tatsächliche Position bzw. Stellung des Kopfs in dem festen Referenzkoordinatensystem erfaßt werden, wozu die Position und Lage, d.h. Ausrichtung, des Kopfs in diesem festen (absoluten) Koordinatensystem erfindungsgemäß vorzugsweise unter Verwendung eines Trägheitsnavigationssystems erfaßt wird.

Unter Trägheitsnavigationssystem sollen erfindungsgemäß solche Systeme verstanden werden, die in der Lage sind, in der Regel ausgehend von einem definierten Anfangszustand, die Kopfposition relativ zu dem festen Referenzkoordinatensystem zu ermitteln. Unter Kopfposition ist dabei sowohl der räumliche Aufenthaltsort des Kopfes, bzw. eines Referenzpunkts desselben, als auch die Ausrichtung des Kopfes bezüglich des festen Koordinatensystems zu verstehen. Die vorliegende Erfindung schafft somit Vorrichtungen und Verfahren zur absoluten Blickrichtungsbestimmung mit Inertial- und EOG-Signalen.

Im einfachsten Fall umfaßt das Trägheitsnavigationssystem Mittel zum Erfassen von Beschleunigungen in zumindest drei zueinander senkrechten Richtungen, die den Achsen eines kartesischen Koordinatensystems entsprechen. Vorzugsweise kann das Trägheitsnavigationssystem ferner Mittel zum Erfassen einer Neigung bzw. Drehrate um drei zueinander senkrechte Achsen, die denen des kartesischen Koordinatensystems entsprechen können, aufweisen. Als Trägheitsnavigationssystem kann erfindungsgemäß dabei jegliches bekanntes System verwendet werden, das in der Lage ist, die Position, d.h. den Aufenthaltsort und die Ausrichtung, eines Körpers relativ zu einer festen Referenz zu bestimmen.

Ist die Augenblickrichtung relativ zum Kopf des Benutzers und die Kopfposition relativ zu dem Referenzkoordinatensystem bekannt, kann daraus ohne weiteres unter Verwendung vektorieller Betrachtungen die Blickrichtung relativ zu dem Referenzkoordinatensystem, d.h. die absolute Augenblickrichtung, unter Verwendung einer geeigneten Vorrichtung, beispielsweise eines Mikroprozessors, bestimmt werden.

Die derart ermittelte absolute Blickrichtung kann für eine Vielzahl von Anwendungen vorteilhaft genutzt werden. Beispielsweise kann in VR-Anwendungen (VR = Virtual Reality) die Steuerung von Szenen abhängig von der absoluten Blickrichtung des Benutzers, beispielsweise einer 3D-Brille, gesteuert werden. Ferner kann eine Mensch-Maschine-Schnittstelle vorteilhaft unter Ausnutzung der erfaßten absoluten Blickrichtung realisiert werden, da beispielsweise ein Mauszeiger ohne weiteres exakt abhängig davon gesteuert werden kann, wo ein Benutzer tatsächlich hinblickt. Gemäß dem Stand der Technik erfolgte eine solche Steuerung entweder abhängig von nur einer Augenbewegung oder nur einer Kopfbewegung. Die vorliegende Erfindung ermöglicht in diesem Zusammenhang eine Mensch-Maschine-Schnittstelle mit einer erhöhten Flexibilität und mit einem erhöhten Komfort für den Benutzer. Hierbei kann die vorliegende Erfindung insbesondere auch in Kommunikationshilfen für Behinderte Anwendung finden.

Darüberhinaus ist die vorliegende Erfindung auf dem Gebiet der Augenmeßtechnik zu medizinischen Zwecken anwendbar. Ferner kommen Anwendungen im Kfz-Bereich und dergleichen in Betracht.

Weiterbildungen der vorliegenden Erfindung sind in den abhängigen Ansprüchen dargelegt.

Bevorzugte Ausführungsbeispiele der vorliegenden Erfindung werden nachfolgend bezugnehmend auf die beiliegenden Zeichnungen näher erläutert. Es zeigen:

Fig. 1    eine schematische Darstellung zur Erläuterung von relativer Augenblickrichtung und absoluter Augenblickrichtung; und

Fig. 2 ein bevorzugtes Ausführungsbeispiel der vorliegenden Erfindung bei Verwendung als Mensch-Maschine-Schnittstelle.

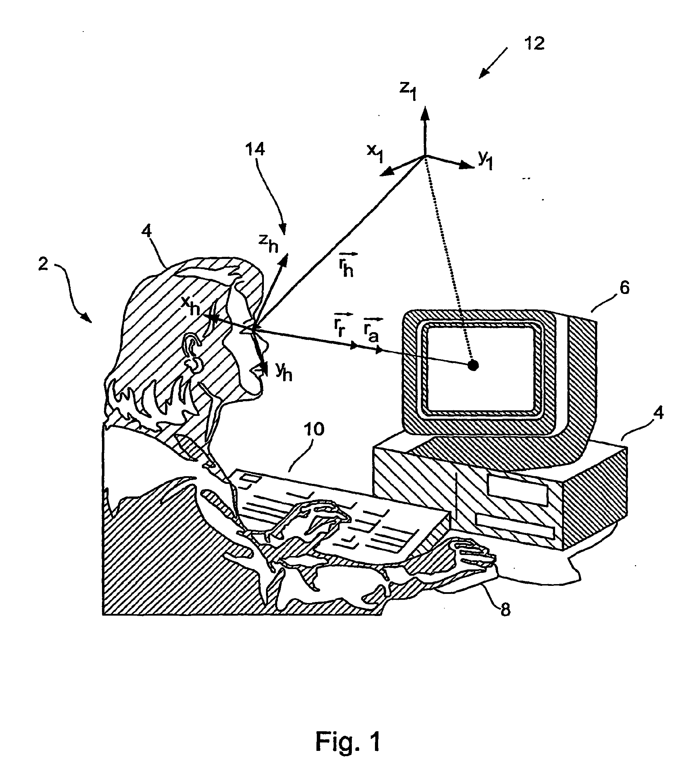

Bezugnehmend auf Fig. 1 wird nachfolgend der Zusammenhang zwischen relativer Blickrichtung, d.h. Blickrichtung der Augen relativ zum Kopf des Benutzers, und absoluter Blickrichtung, d.h. Blickrichtung relativ zu einem festen Referenzkoordinatensystem, erläutert.

Zu diesem Zweck ist in Fig. 1 ein Benutzer bzw. eine Benutzerin 2 gezeigt, die sich vor einem Computer 4 mit einem zugehörigen Bildschirm 6 befindet. Ferner ist der Computer in Fig. 1 mit einer herkömmlichen Mensch-Maschine-Schnittstelle in der Form einer Maus 8 und einer Tastatur 10 ausgestattet.

Ferner ist in Fig. 1 ein festes Referenzkoordinatensystem 12 mit den Koordinatenachsen $x_f$, $y_f$ und $z_f$ und ein dem Kopf 4 der Benutzerin zugeordnetes und somit bewegliches Koordinatensystem 14 mit den drei zueinander senkrechten Koordinatenachsen $x_h$, $z_h$ und $y_h$ gezeigt. Der Blick der Benutzerin 2 ist auf einen Fixpunkt 16 auf dem Bildschirm 6 gerichtet. Die dadurch bedingte Blickrichtung der Benutzerin ist durch den Vektor $\overline{r}_r$ in Fig. 1 gezeigt. Ferner ist in Fig. 1 die absolute Blickrichtung der Benutzerin 2 durch den Vektor $\overline{r}_a$ angezeigt. Sowohl $\overline{r}_a$ als auch $\overline{r}_r$ weisen bezüglich des festen Koordinantensystems 12 die gleiche Richtung auf. Dabei ist die Blickrichtung $\overline{r}_r$, die erfindungsgemäß vorzugsweise auf der Grundlage eines Elektrookulogramms bestimmt wird, auf das Koordinatensystem 14 des Kopfs 4 der Benutzerin 2 bezogen und nicht auf das feste Referenzkoordinatensystem 12. Ferner ist in Fig. 1 die absolute Kopfposition der Benutzerin 2 durch den Vektor $\overline{r}_h$ angezeigt, d.h. die Position relativ zu dem festen Referenzkoordinatensystem 12 bzw. dem Ursprung desselben.

Die Lage des Kopfkoordinatensystems 14 bezüglich des festen Referenzkoordinatensystems 12 hängt von der Position bzw. Stellung des Kopfs 4 bezüglich des Referenzkoordinatensystems 12 ab. Wenn daher die relative Blickrichtung $\overline{r}_r$ und die Kopfposition erfaßt wird, kann ohne weiteres die absolute Blickrichtung, d.h. die Blickrichtung bezüglich des festen Koordinatensystems basierend auf vektoriellen Betrachtungen ermittelt werden. Zu diesem Zweck muß ausgehend von der erfaßten Relativblickrichtung, die auf das Kopfkoordinatensystem 14 bezogen ist, lediglich die Stellung der beiden Koordinantensysteme 12 und 14 zueinander berücksichtigt werden, die sich aus der Kopfposition bezüglich des festen Referenzkoordinatensystems ergibt. Somit kann die absolute Blickrichtung der Benutzerin, die auf das feste Referenzkoordinantensystem 12 bezogen ist, zu jedem Zeitpunkt ohne weiteres ermittelt werden, solange die Position des Kopfs 4 bezüglich des festen Referenzkoordinatensystems bekannt ist. Unter Position ist dabei sowohl die räumliche Lage des Kopfs als auch die Ausrichtung, d.h. die Neigung, Drehung usw., desselben bezüglich des Referenzkoordinatensystems zu verstehen.

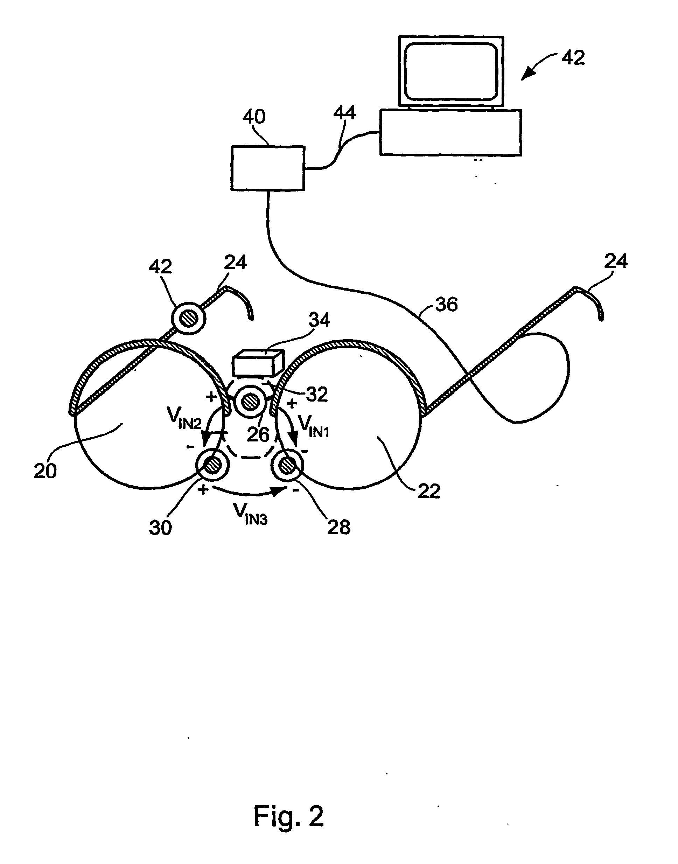

Ein bevorzugtes Ausführungsbeispiel einer erfindungsgemäßen Vorrichtung zur Blickrichtungsbestimmung bezüglich eines festen Referenzkoordinatensystems, d.h. zur absoluten Blickrichtungsbestimmung, ist in Fig. 2 gezeigt und kann als EOG-Brille bezeichnet werden. Diese Vorrichtung umfaßt einen Aufbau, der im wesentlichen dem einer bekannten Brille entspricht, jedoch keine Brillengläser aufweisen muß. Optional können Brillengläser 20 und 22 für das rechte bzw. linke Auge eines Benutzers vorgesehen sein, wie es in Fig. 2 angedeutet ist, um bei Bedarf Sehmängel eines Benutzers auszugleichen.

Die EOG-Brille ist wie eine Brille tragbar und umfaßt zu diesem Zweck Bügel 24. Ferner sind Elektroden 26, 28, 30 vorgesehen, mittels derer die EOG-Brille bei bevorzugten Ausführungsbeispielen auf der Nase eines Benutzers aufsitzt. Somit ist die Vorrichtung derart konstruiert, daß bereits das Aufsetzen einen guten Kontakt zwischen Haut und Elektroden, der zur Aufnahme von EOG-Signalen erforderlich ist, gewährleistet. Ferner ist dadurch gewährleistet, daß beim Tragen der EOG-Brille keine Relativbewegungen zwischen derselben und dem Kopf des Benutzers auftreten. Durch die optionale Anwendung von Druckknopfkontakten können auch handelsübliche Einmalelektroden zwecks gegebenenfalls erforderlicher Hygienemaßnahmen zum Einsatz kommen.

Über die Elektroden, die auf der Hautoberfläche des Benutzers bzw. der Benutzerin aufliegen, können die durch elektrische Dipolfelder erzeugten Biosignale abgegriffen werden, indem die in Fig. 2 dargestellten Spannungen $V_{IN1}$, $V_{IN2}$ und $V_{IN3}$ meßtechnisch erfaßt werden. Zu diesem Zweck ist die Vorrichtung mit geeigneten Vorverstärkern ausgestattet, die in Fig. 2 durch eine gestrichelte Linie 32 angedeutet sind, so daß möglichst kurze Leitungen zu den Vorverstärkern realisiert sein können, wodurch Störsignaleinflüsse minimiert und die Signalqualität verbessert werden kann.

Bezüglich dieser Anordnung zur relativen Blickrichtungserfassung sei auf die oben genannte Schrift von P. Wiebe, "Biosignalverarbeitung des menschlichen Elektrookulogramms (EOG) zur Steuerung von Computer-Eingabemedien für gelähmte Menschen" verwiesen.

Die in Fig. 2 dargestellte erfindungsgemäße Vorrichtung umfaßt ferner eine Vorrichtung zur Erfassung der Kopfposition des Benutzers, der die EOG-Brille trägt, bezüglich eines festen Referenzkoordinatensystems, die im folgenden als Trägheitsnavigationssystem bezeichnet wird.

Die abgegriffenen Spannungen $V_{IN1}$, $V_{IN2}$ und $V_{IN3}$ sowie die Ausgangssignale des Trägheitsnavigationssystems 34, die ebenfalls in geeigneter Weise vorverstärkt werden können, werden über eine Leitung 36 in geeigneter Form einer Verarbeitungseinrichtung 40 zugeführt, die auf der Grundlage dieser Signale die absolute Blickrichtung des Benutzers, der die EOG-Brille trägt, d.h. die Blickrichtung bezüglich des festen Referenzkoordinatensystems, ermittelt. An dem in Fig. 2 rechten Bügel der brillenartigen Vorrichtung ist ferner schematisch eine Masseelektrode 42 dargestellt, die aber auch beliebige andere geeignet Orte der Kopfhautoberfläche einnehmen kann.

Sollen auf der Grundlage der erfaßten absoluten Blickrichtung die Funktionen einer herkömmlichen Computermaus realisiert werden, ist die Verarbeitungseinrichtung 40 in geeigneter Form mit einem Computer, der in Fig. 2 schematisch bei 42 gezeigt ist, über eine Leitung 44 gekoppelt. Es ist klar, daß die in Fig. 2 gezeigten Leitungen 36 und 44 in geeigneter Weise durch drahtlose Übertragungsmechanismen ersetzt werden können.

Um auf der Grundlage der über die Elektroden 26, 28 und 30 abgegriffenen Spannungen $V_{IN1}$, $V_{IN2}$ und $V_{IN3}$ die relative Blickrichtung $\bar{r}_r$ (Fig. 1) des Benutzers zu ermitteln, werden die Spannungen zunächst, wie oben angegeben wurde, unter Verwendung geeigneter Vorverstärker 32 einer Vorverstärkung unterzogen. Die so verstärkten erfaßten Spannungen werden zur Verarbeitungseinrichtung 40 übertragen, in der optional eine Mittelwertbildung aus jeweils einer vorbestimmten Anzahl von Abtastwerten durchgeführt wird, um eine Verringerung der Rauschanteile zu bewirken. Die somit auf der Grundlage der Spannungen $V_{IN1}$ und $V_{IN2}$ enthaltenen Signale bilden die Grundlage für das vertikale EOG, indem vorzugsweise eine richtungsbezogene Addition dieser Signale durchgeführt wird, wodurch sich die beiden horizontalen Komponenten bei exakt symmetrischer Dipolfeld-Verteilung vollständig aufheben, so daß das vertikale EOG vom horizontalen EOG weitgehend entkoppelt werden kann. Ferner verdoppelt sich durch diese Addition die Amplitude des vertikalen EOG. Als horizontales EOG wird das auf der Grundlage der Spannung $V_{IN3}$ gewonnene Ausgangssignal verwendet.

Aus den so ermittelten EOG-Signalverläufen, die das Dipolfeld des Augapfels und somit Bewegungen desselben bzw. die Stellung desselben bezüglich des Kopfes anzeigen, kann somit die Augenblickrichtung relativ zum Kopf erfaßt werden.

Das Trägheitsnavigationssystem 34 besteht im einfachsten Fall aus einem oder mehreren Beschleunigungssensoren, der oder die in der Lage sind, Beschleunigungen in den drei Richtungen der Achsen eines kartesischen Koordinatensystems zu erfassen. Vorzugsweise weist das Trägheitsnavigationssystem ferner Neigungs- bzw. Drehraten-Sensoren auf, um Drehungen bzw. Neigungen um die drei Achsen zu erfassen. Basierend auf den Ausgangssignalen dieser Sensoren kann ausgehend von einem Anfangszustand, der beispielsweise unter Verwendung einer geeigneten Kalibrierung des Trägheitsnavigationssystems bezüglich des Referenzkoordinatensystems ermittelt wird, und ferner unter Berücksichtigung der konstanten Erdbeschleunigung die Kopfposition im Raum bestimmt werden, da zum einen die Geschwindigkeit v das Integral der Beschleunigung a über der Zeit t und zum anderen die Position r das Integral der Geschwindigkeit über der Zeit ist.

Aus den erfaßten Beschleunigungen a ist somit basierend auf folgender Gleichung die Kopfposition r ermittelbar:

$$\bar{r}_h \;=\; \iint \left\{ \bar{a}_x(t) \,+\, \bar{a}_y(t) \,+\, \bar{a}_z(t) \right\} dt\, dt$$

Somit kann in bekannter Weise aus dem in die drei Richtungen des kartesischen Koordinatensystems erfaßten Beschleunigungen die Kopfposition durch entsprechende Berechnungen ermittelt werden. Zur Durchführung derartiger Berechnungen und ferner zur Signalverarbeitung der EOG-Signale kann spezielle Hardware und/oder Software verwendet werden.

Aus den erfaßten EOG-Signalen und den Ausgangssighälen des Trägernavigationssystems kann dann in der Verarbeitungseinrichtung 40 die absolute Blickrichtung, d.h. die Blickrichtung bezüglich eines festen Koordinatensystems bestimmt werden. Beispielsweise kann die z-Achse des festen Koordinatensystems die Vertikale bezüglich der Erdoberfläche sein, während die x-Achse und die y-Achse in der Horizontalen liegen, d.h. die horizontale Ebene aufspannen.

Aus der somit ermittelten absoluten Blickrichtung können dann in der Verarbeitungseinrichtung 40 entsprechende Ansteuersignale zum Bewegen eines Cursors auf dem Bildschirm des Computers 42 generiert werden. Daneben können Lidbewegungen, die ebenfalls über die Elektroden 26, 28 und 30 erfaßt werden, in entsprechende Eingabesignale umgewandelt werden, wobei bestimmte willentliche Lidbewegungen beispielsweise als linker Mausklick oder rechter Mausklick aufgefaßt werden können. Die Ausgangssignale der Verarbeitungseinrichtung 40 können in einem solchen Fall aufbereitet werden, um analog zu denen zu sein, die von einer herkömmlichen Maus erzeugt werden.

Die vorliegende Erfindung zur Erfassung der absoluten Blickrichtung ermöglicht somit eine Mensch-Maschine-Schnittstelle basierend auf den Biosignalen "relative Blickrichtung", "Lidbewegung" und "Kopfbewegung". Die dazu geforderte absolute Blickrichtung wird vorzugsweise durch die Kombination der Gewinnung elektrookulographischer Signale der Augen und der unter Verwendung eines Trägheitsnavigationssystems ermittelten Positionsdaten des Kopfes bestimmt. Auf diese Weise können zahlreiche nützliche Mensch-Maschine-Schnittstellen, die die absolute Blickrichtung des Anwenders bzw. Benutzers erfordern, realisiert werden.

**Patentansprüche**

1.  Vorrichtung zur Blickrichtungsbestimmung bezüglich eines festen Referenzkoordinatensystems (12), mit folgenden Merkmalen:

    einer Einrichtung (26, 28, 30, 32, 40) zur Erfassung von Elektrookulogrammen, um die Augenblickrichtung eines Benutzers (2) relativ zum Kopf (4) des Benutzers zu erfassen;
    einem Trägheitsnavigationssystem zur Erfassung der Position des Kopfes (4) relativ zu dem festen Referenzkoordinatensystem (12); und
    einer Einrichtung zur Ermittlung der Augenblickrichtung des Benutzers (2) bezüglich des festen Referenzkoordinatensystems (12) aus der erfaßten Blickrichtung relativ zum Kopf (4) und der erfaßten Position des Kopfs (4) relativ zu dem festen Referenzkoordinatensystem (12).

2.  Vorrichtung nach Anspruch 1, bei der die Einrichtung zur Erfassung von Elektrookulogrammen auf einer in der Art einer Brille durch einen Benutzer tragbaren Vorrichtung angeordnet ist und zumindest drei Elektroden (26, 28, 30) zum Erfassen von mindestens zwei Spannungen basierend auf Augendipolfeldern aufweist.

3.  Vorrichtung nach Anspruch 1, bei der das Trägheitsnavigationssystem auf der in der Art einer Brille tragbaren Vorrichtung angeordnet ist.

4.  Vorrichtung nach einem der Ansprüche 1 bis 3, bei der das Trägheitsnavigationssystem (34) Mittel zum Erfassen von Beschleunigungen in zumindest drei zueinander senkrechten Richtungen umfaßt.

5.  Vorrichtung nach Anspruch 4, bei der das Trägheitsnavigationssystem (34) ferner Mittel zum Erfassen einer Drehung um zumindest drei zueinander senkrechte Achsen umfaßt.

6.  Verfahren zur Bestimmung der Blickrichtung bezüglich eines festen Referenzkoordinatensystems (12), mit folgenden Schritten:

    Messen des Dipolfelds der Augen eines Benutzers (2), um die Augenblickrichtung des Benutzers (2) relativ zum Kopf (4) des Benutzers zu erfassen;
    Erfassen von Inertial-Signälen, um die Position des Kopfs (4) des Benutzers (2) relativ zu dem festen Referenzkoordinatensystem (12) zu erfassen; und
    Bestimmen der Augenblickrichtung des Benutzers (2) relativ zu dem festen Referenzkoordinatensystem (12) aus der erfaßten Augenblickrichtung des Benutzers (2) relativ zum Kopf (4) des Benutzers und der erfaßten Position des Benutzers (2) relativ zu dem festen Referenzkoordinatensystem (12).

7.  Verfahren nach Anspruch 6, bei dem die Inertial-Signale durch Beschleunigungsmessungen und Drehratenmessungen erfasst werden.


**Claims**

1.  A device for determining the viewing direction relative to a fixed reference co-ordinate system (12), comprising:

    means (26, 28, 30, 32, 40) for detecting electrooculograms for detecting the viewing direction of the eyes of a user (2) relative to the user's head (4);
    an inertial navigation system for detecting the position of the head (4) relative to said fixed reference co-ordinate system (12); and
    means for determining the viewing direction of the eyes of the user (2) relative to said fixed reference co-ordinate system (12) from the detected viewing direction relative to the head (4) and from the detected position of the head (4) relative to said fixed reference co-ordinate system (12).

2.  A device according to claim 1, wherein the means for detecting electrooculograms is arranged on a device that can be worn by a user like spectacles, and comprises at least three electrodes (26, 28, 30) for detecting at least two voltages on the basis of eye dipole fields.

3.  A device according to claim 1, wherein said inertial navigation system is arranged on the device that can be worn like spectacles.

**4.** A device according to one of the claims 1 to 3, wherein said inertial navigation system (34) comprises means for detecting accelerations in at least three mutually perpendicular directions.

**5.** A device according to claim 4, wherein the inertial navigation system (34) additionally comprises means for detecting a rotation about at least three mutually perpendicular axes.

**6.** A method of determining the viewing direction relative to a fixed reference co-ordinate system (12), said method comprising the following steps:

measuring the dipole field of the eyes of the user (2) to detect the viewing direction of the eyes of the user (2) relative to the head (4) of the user;
detecting inertial signals to detect the position of the head (4) of the user (2) relative to the fixed reference co-ordinate system (12); and
determining the viewing direction of the eyes of the user (2) relative to the fixed reference co-ordinate system (12) from the detected viewing direction of the eyes of the user (2) relative to the head (4) of the user and from the detected position of the user (2) relative to said fixed reference co-ordinate system (12).

**7.** A method according to claim 6, wherein the inertial signals are detected by acceleration measurements and rotary speed measurements.

**Revendications**

**1.** Dispositif pour déterminer la direction du regard par rapport à un système de coordonnées de référence fixe (12), aux caractéristiques suivantes :

un dispositif (26, 28, 30, 32, 40) destiné à détecter des électro-oculogrammes, afin de détecter la direction de regard des yeux d'un utilisateur (2) par rapport à la tête (4) de l'utilisateur ;
un système de navigation par inertie destiné à détecter la position de la tête (4) par rapport au système de coordonnées de référence fixe (12) ; et
un dispositif destiné à déterminer la direction de regard des yeux de l'utilisateur (2) par rapport au système de coordonnées de référence fixe (12) à partir de la direction du regard détectée par rapport à la tête (4) et de la position de la tête détectée (4) par rapport au système de coordonnées de référence fixe (12).

**2.** Dispositif selon la revendication 1, dans lequel le dispositif destiné à détecter des électro-oculogrammes est disposé sur un dispositif pouvant être porté à la manière de lunettes par un utilisateur et présente au moins trois électrodes (26, 28, 30) destinées à capter au moins deux tensions sur base de champs de dipôle des yeux.

**3.** Dispositif selon la revendication 1, dans lequel le système de navigation par inertie est disposé sur le dispositif pouvant être porté à la manière de lunettes.

**4.** Dispositif selon l'une des revendications 1 à 3, dans lequel le système de navigation par inertie (34) comporte des moyens destinés à capter des accélérations dans au moins trois directions perpendiculaires entre elles.

**5.** Dispositif selon la revendication 4, dans lequel le système de navigation par inertie (34) comporte, par ailleurs, des moyens destinés à capter une rotation autour d'au moins trois axes perpendiculaires entre eux.

**6.** Procédé pour déterminer la direction du regard par rapport à un système de coordonnées de référence fixe (12), aux étapes suivantes consistant à :

mesurer le champ de dipôle des yeux d'un utilisateur (2), pour détecter la direction de regard des yeux de l'utilisateur (2) par rapport à la tête (4) de l'utilisateur;
détecter des signaux d'inertie, pour détecter la position de la tête (4) de l'utilisateur (2) par rapport au système de coordonnées de référence fixe (12) ; et
déterminer la direction de regard des yeux de l'utilisateur (2) par rapport au système de coordonnées de référence fixe (12) à partir de la direction de regard détectée des yeux de l'utilisateur (2) par rapport à la tête (4) de l'utilisateur et de la position détectée de l'utilisateur (2) par rapport au système de coordonnées de référence fixe (12).

7. Procédé selon la revendication 6, dans lequel les signaux d'inertie sont détectés par des mesures d'accélération et des mesures de taux de rotation.

Fig. 1

Fig. 2